# EUROPÄISCHE PATENTSCHRIFT

(11) **EP 1 678 143 B1**
(45) Veröffentlichungstag und Bekanntmachung des Hinweises auf die Patenterteilung: **29.06.2011**
(21) Anmeldenummer: 04790292.9
(22) Anmeldetag: 12.10.2004
(51) Int. Cl.: C07D 231/16

(54) **N-SUBSTITUIERTE PYRAZOLYLCARBOXANILIDE**
N-SUBSTITUTED PYRAZOLYL CARBOXANILIDES
PYRAZOLYLCARBOXANILIDES N-SUBSTITUES

(30) Priorität: 23.10.2003 DE 10349497
(43) Veröffentlichungstag der Anmeldung: 12.07.2006
(73) Patentinhaber: Bayer CropScience Aktiengesellschaft, 40789 Monheim (DE)
(72) Erfinder: DUNKEL, Ralf, 69001 Lyon (FR); ELBE, Hans-Ludwig, 42329 Wuppertal (DE); GREUL, Jörg, Nico, 42799 Leichlingen (DE); HARTMANN, Benoit, 40764 Langenfeld (DE); WACHENDORFF-NEUMANN, Ulrike, 56566 Neuwied (DE); DAHMEN, Peter, 41470 Neuss (DE); KUCK, Karl-Heinz, 40764 Langenfeld (DE)
(86) Internationale Anmeldenummer: PCT/EP2004/011396
(87) Internationale Veröffentlichungsnummer: WO 2005/042480

(56) Entgegenhaltungen:
- EP-A- 0 545 099
- WO-A-02/059086
- WO-A-2004/103975

## Beschreibung

Die vorliegende Erfindung betrifft neue (N-substituierte) Pyrazolylcarboxanilide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vgl. WO 93/11117, EP-A 0 545 099, EP-A 0 824 099, JP 63-48269, WO 02/059086 und JP 8-176112). Beispielhaft seien N-[2-(1,3-Dimethylbutyl)phenyl]-5-fkuor-1-methyl-N-prop-2-in-1-yl-3-(trifluormethyl)-1H-pyrazol-4-carboxamid und N-Acetyl-N-[2-(1,3-dimethylbutyl)phenyl]-5-fluor-1-methyl-3-(trifluormethyl)-1H-pyrazol-4-carboxamid genannt (WO 02/059086). Die Wirksamkeit dieser Stoffe ist gut, lässt aber bei niedrigen Aufwandmengen in manchen Fällen zu wünschen übrig.

Es wurden nun neue N-substituierte Pyrazolylcarboxanilide der Formel (I) 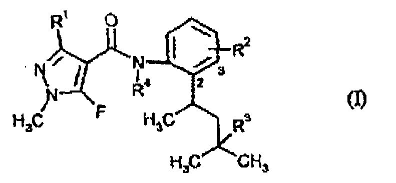 gefunden, in welcher
- R¹: für Methyl steht,
- R²: für Wasserstoff, Fluor, Chlor, Methyl oder Trifluormethyl steht,
- R³: für Wasserstoff oder für Halogen, C₁-C₈-Alkyl oder C₁-C₈-Halogenalkyl steht,
- R⁴: für C₁-C₈-Alkyl, C₁-C₄-Alkoxy-C₁-C₄-al kyl, oder -C(O=)C(=O)R⁵ steht,
- R⁵: für Wasserstoff, C₁-C₈-Alkyl, C₁-C₈-Alkoxy, C₁-C₄-Alkoxy-C₁-C₄-alkyl, C₃-C₈-Cycloalkyl;, C₁-C₆-Halogenalkyl, C₁-C₆-Halogenalkoxy, Halogen-C₁-C₄-alkoxy-C₁-C₄-alkyl, C₃-C₈-Halo- gencycloalkyl mit jeweils 1 bis 9 Fluor-, Chlor- und/oder Bromatomen steht.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenerefalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Weiterhin wurde gefunden, dass man N-substituierte Pyrazolylcarboxanilide der Formel (I) erhält, indem man
a) Carbonsäure-Derivate der Formel (II) 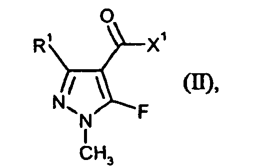 in welcher
   - R¹: die oben angegebenen Bedeutungen hat und
   - X¹: für Halogen oder Hydroxy steht,
   mit einem Anilinderivat der Formel (III) 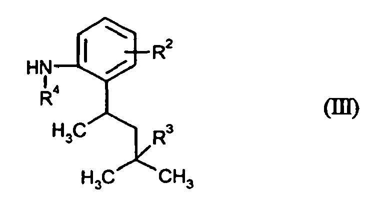 in welcher R², R³ und R⁴ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
   oder
b) Pyrazolylcarboxanilide der Formel (IV) 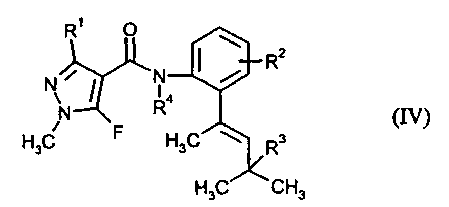 in welcher R¹, R², R³ und R⁴ die oben angegebenen Bedeutungen haben,
   gegebenenfalls in Gegenwart eines Verdünnungsmittels und gegebenenfalls in Gegenwart eines Katalysators hydriert,
   oder
c) Pyrazolylcarboxanilide der Formen (Ia) 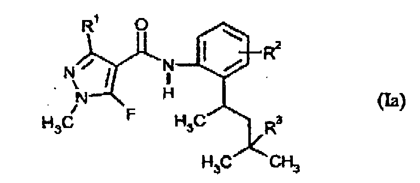 in welcher R¹, R² und R³ die oben angegebenen Bedeutungen haben
   mit Halogeniden der Formel (V)

   R⁴-X² (V)

   in welcher
   - R⁴: die oben angegebenen Bedeutungen hat und
   - X²: für Chlor, Brom oder Iod steht,
   in Gegenwart einer Base und in Gegenwart eines Verdünnungsmittels umsetzt.

Schließlich wurde gefunden, dass die neuen N-substituierten Pyrazolylcarboxanilide der Formel (I) sehr gute mikrobizide Eigenschaften besitzen und zur Bekämpfung unerwünscher Mikroorganismen sowohl im Pflanzenschutz als auch im Materialschutz verwendbar sind.

Die erfindungsgemäßen N-substituierten Pyrazolylcarboxamilide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹: Steht für für Methyl,
- R²: steht bevorzugt für Wasserstoff:
- R²: steht außerdem bevorzugt für Fluor, wobei Fluor besonders bevorzugt in, 4-, 5- oder 6- Position, ganz besonders bevorzugt in 4- oder 6-Position, insbesondere in 4-Position des Anilidrestes steht [vgl. oben Formel (I)].
- R²: steht außerdem bevorzugt für Chlor, wobei Chlor besonders bevorzugt in 5-Position des Anilidrestes steht [vgl. oben Formel (I)].
- R²: steht außerdem bevorzugt für Methyl, wobei Methyl besonders bevorzugt in 3-Position des Anilidrestes steht [vgl. oben Formel (I)].
- R²: steht außerdem bevorzugt für Trifluormethyl, wobei Trifluormethyl besonders bevorzugt in 4- oder 5-Position des Anilidrestes steht [vgl. oben Formel (I)].

Wenn R³ für Wasserstoff steht, dann steht
- R⁴: bevorzugt für C₁₋C₆-Alkyl, C₁-C₃-Alkoxy-C₁-C₃- alkyl, oder -C(=O)C(=O)R⁵

Wenn R³ für Wasserstoff steht, dann steht
- R⁴: steht besonders bevorzugt für Methyl, Ethyl, n- oder iso-Propyl, n-, iso-, sec- oder tert-Butyl, Pentyl oder Hexyl,

Wenn R³ für Wasserstoff steht, dann steht
- R⁴: steht ganz besonders bevorzugt für Methyl, Methoxymethyl, -C(=O)C(=O)CH₃, -C(=O)C(=O)CH₂OCH₃,

Wenn
- R³: bevorzugt für Fluor, Chlor, Brom, Iod, C₁-C₆-Alkyl oder C₁-C₆-Halogenalkyl mit 1 bis 13 Fluor-, Chlor- und/oder Bromatomen steht, besonders bevorzugt für Fluor, Chlor, Brom, Methyl, Ethyl, n-, iso-Propyl, n-, iso-, sec-, tert- Butyl oder C₁-C₄-Halogenalkyl mit 1 bis 9 Fluor-, Chlor- und/oder Bromatomen steht, ganz, besonders, bevorzugt für Methyl, Ethyl, Fluor, Chlor, Trifluormethyl steht, dann steht
- R⁴: bevorzugt für C₁-C₆-Alkyl, C₁-C₃-Alkoxy-C₁-C₃- alkyl; oder -C(=O)C(=Q)R⁵, besonders bevorzugt für Methyl, Ethyl, n- oder iso-Propyl, n-, iso-, sec- oder tert-Butyl, Pentyl oder Hexyl, oder -C(=O)C(+O)R⁵ ganz besonders bevorzugt für Methyl, Methoxymethyl, -C(=O)C(=O)CH₃, -C(=O)C(=O)CH₂OCH₃,
- R⁵ 1: steht bevorzugt für Wasserstoff, C₁-C₆-Alkyl, C₁-C₄-Alkoxy, C₁-C₃-Alkoxy-C₁-C₃-alkyl C₃- C₆-Cycloalkyl; C₁-C₄-Halogenalkyl, C₁-C₄-Halegenalkoxy, Halogen-C₁-C₃-alkoxy-C₁-C₃- alkyl, C₃-C₆-Halogencycloalkyl mit jeweils 1 bis 9 Fluor-, Chlor- und/oder Bromatomen.
- R⁵: steht besonders bevorzugt für Wasserstoff, Methyl, Ethyl, n- oder iso-Propyl, tert-Butyl, Methoxy, Ethoxy, n- oder iso-Propoxy, tert-Butoxy, Cyclopropyl; Trifluormethyl, Trifluor- methoxy.

Hervorgehoben sind Verbindungen der Formel (I), in welcher R⁴ für -C(=O)C(=O)R⁵ steht, wobei R⁵ die oben angegebenen Bedeutungen hat.

Hervorgehoben sind weiterhin N-substituierte Pyrazolylcarboxanilide der Formel (Ib) 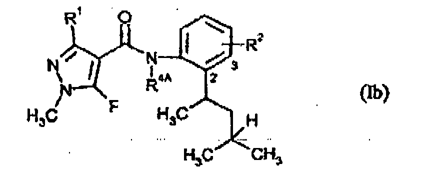 in welcher
- R^{4A} Für: C₁-C₈-Alkyl, C₁-C₄-Alkoxy-C₁-C4-alkyl, oder -C(=O)C(=O)R⁵, steht, und R¹, R² und R⁵ die oben angegebenen Bedeutungen halben.

Hervorgehoben sind weiterhin N-substituierte Pyrazolylcarboxanilide der Formel (Ic) 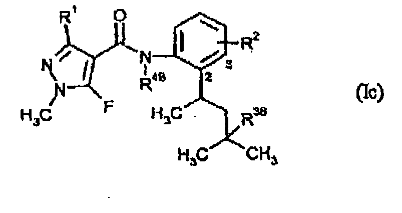 in welcher
- R^{3B}: für Halogen, C₁-C₈-Alkyl oder C₁-C₈-Halogenalkyl steht,
- R^{4B}: für C₁-C₈-Alkyl, C₁-C₄-Alkoxy-C₁-C₄-alkyl; oder -C(=O)C(=O)R⁵ steht,
und R¹, R² und R⁵ die oben angegebenen Bedeutungen haben.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein. Gegebenenfalls substituierte Reste können einfach oder mehrfach substituiert sein, wobei bei Mehrfachsubstitutionen die Substituenten gleich oder verschieden sein können.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die genannten Definitionen können untereinander in beliebiger Weise kombiniert werden. Außerdem können auch einzelne Definitionen entfallen.

Bevorzugt, besonders bevorzugt oder ganz besonders bevorzugt sind Verbindungen der Formel (I), welche jeweils die unter bevorzugt, besonders bevorzugt oder ganz besonders bevorzugt genannten Substituenten tragen.

Verwendet man 5-Fluor-1,3-dimethyl-1H-pysazol-4-carbonyl-chlorid und N-(Methoxymethyl)-2-(1,3,3-trimethylpentyl)anilin als Ausgangsstoffe, so kann das erfindungsgemäße Verfahren (a) durch das folgende Formelschema veranschaulicht werden:

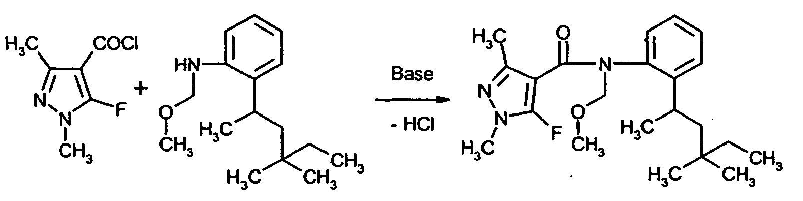

Die zur Durchführung des erfindungsgemäßen Verfahrens (a) als Ausgangsstoffe benötigten Carbonsäure-Derivate sind durch die Formel (II) allgemein definiert. In dieser Formel (II) hat R¹ bevorzugt diejenigen Bedeutungen, die bereits im Zusammenhang mit der Beschreibung der erfindungsgemäßen Verbindungen der Formel (I) als bevorzugt für R¹ angegeben wurden. X¹ steht bevorzugt für Chlor, Brom oder Hydroxy.

Die Carbonsäure-Derivate der Formel (II) sind bekannt und/oder lassen sich nach bekannten Verfahren herstellen (vgl. WO 93/11117, EP-A 0 545 099, EP-A 0 589 301 und EP-A 0 589 313).

Die zur Durchführung des erfindungsgemäßen Verfahrens (a) als Ausgangsstoffe weiterhin benötigten Aniline sind durch die Formel (III) allgemein definiert. In dieser Formel (III) haben R², R³ und R⁴ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden.

Die Anilin-Derivate der Formel (III) sind neu. Sie lassen sich herstellen, indem man,
d) Alkylaniline der Formel (VI) 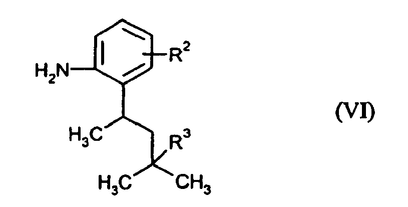 in welcher R² und R³ die oben angegebenen Bedeutungen haben,
   mit Halogeniden der Formel (V)

   R⁴-X² (V)

   in welcher
   - R⁴: die oben angegebenen Bedeutungen hat und
   - X²: für Chlor, Brom oder Iod steht,
gegebenenfalls in Gegenwart einer Base und in Gegenwart eines Verdünnungsmittels umsetzt.

Die zur Durchführung des erfindungsgemäßen Verfahrens (d) als Ausgangsstoffe benötigten Alkylaniline sind durch die Formel (VI) allgemein defmiert. In dieser Formel (VI) haben R² und R³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden..

Die Alkylaniline der Formel (VI) sind bekannt und/oder können nach bekannten Methoden erhalten werden (vgl. WO 03/010149).

Die zur Durchführung des erfindungsgemäßen Verfahrens (d) als Ausgangsstoffe weiterhin benötigten Halogenide sind durch die Formel (V) allgemein definiert. In dieser Formel (V) hat R⁴ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für R⁴ als bevorzugt, besonders bevorzugt bzw. ganz besonders bevorzugt angegeben wurden.

Halogenide der Formen (V) sind bekannt.

Verwendet man N-Acetyl-5-fluor-1,3-dimethyl N-{2-[(1Z)-1,3,3-trimethylbut-1-en-1-yl]phenyl}-1H-pyrazol-4-carboxamid und Wasserstoff als Ausgangsstoffe sowie einen Katalysator, so kann der Verlauf des erfindungsgemäßen Verfahrens (b) durch das folgende Formelschema veranschaulicht werden:

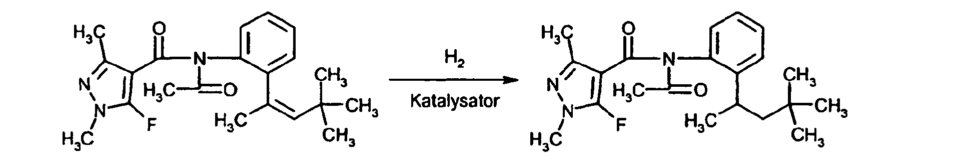

Die zur Durchführung des erfindungsgemäßen Verfahrens (b) als Ausgangsstoffe benötigten Pyrazolylcarboxanilide sind durch die Formel (IV) allgemein definiert. In dieser Formel (IV) haben R¹, R², R³ und R⁴ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Verbindungen der Formel (IV) sind neu. Sie lassen sich herstellen, indem man
e) Pyrazolylcarboxanilide der Formel (VII) 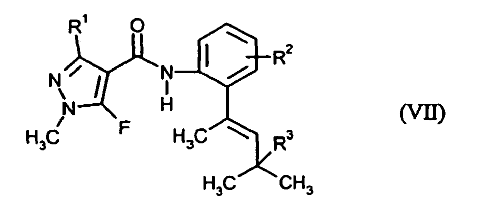 in welcher R¹, R² und R³ die oben angegebenen Bedeutungen haben,
   mit Halogeniden der Formel (V)

   R⁴-X² (V)

   in welcher
   - R⁴: die oben angegebenen Bedeutungen hat und
   - X²: für Chlor, Brom oder Iod steht,
gegebenenfalls in Gegenwart einer Base und in Gegenwart eines Verdünnungsmittels umsetzt.

Die zur Durchführung des erfindungsgemäßen Verfahrens (e) als Ausgangsstoffe benötigten Pyrazolylcarboxanilide sind durch die Formel (VII) allgemein definiert. In dieser Formel (VII) haben R¹, R² und R³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Pyrazolylcarboxanilide der Formel (VII) sind bekannt (vgl. WO 03/010149).

Die zur Durchführung des erfindungsgemäßen Verfahrens (e) als Ausgangsstoffe weiterhin benötigten Halogenide der Formel (V) sind bereits im Zusammenhang mit dem erfindungsgemäßen Verfahren (d) beschrieben worden.

Verwendet man 5-Fluor-1,3-dimethyl-N-[2-(1,3,3-trimethylbutyl)phenyl]-1H-pyrazol-4-carboxamid und Ethyl-chlor(oxo)acetat als Ausgangsstoffe, so kann der Verlauf des erfindungsgemäßen Verfahrens (c) durch das folgende Formelschema veranschaulicht werden:

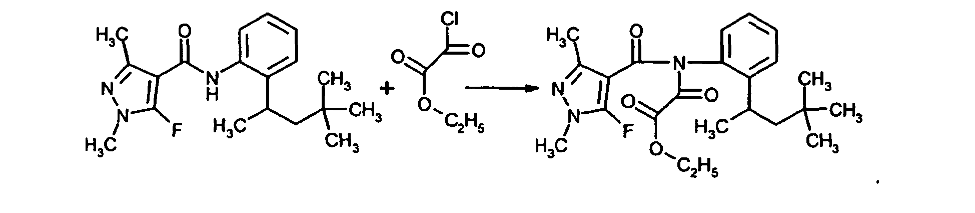

Die zur Durchführung des erfindungsgemäßen Verfahrens (c) als Ausgangsstoffe benötigten Pyrazolylcarboxanilide sind durch die Formel (Ia) allgemein definiert. In dieser Formel (Ia) haben R¹, R² und R³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Pyrazolylcarboxanilide der Formel (Ia) sind bekannt (vgl. WO 03/010149).

Die zur Durchführung des erfindungsgemäßen Verfahrens (c) als Ausgangsstoffe weiterhin benötigten Halogenide der Formel (V) sind bereits im Zusammenhang mit dem erfindungsgemäßen Verfahren (d) beschrieben worden.

Als Verdünnungsmittel zur Durchführung des erfindungsgemäßen Verfahrens (a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäßes Verfahren (a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro mol des Carbonsäure-Derivates der Formel (II) im allgemeinen 0,2 bis 5 mol, vorzugsweise 0,5 bis 2 mol an Anilinderivat der Formel (III) ein.

Als Verdünnungsmittel zur Durchführung des erfindungsgemäßen Verfahrens (b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol oder Alkohole, wie Methanol, Ethanol, n- oder i-Propanol, n-, i-, sec- oder tert-Butanol, Ethandiol, Propan-1,2-diol, Ethoxyethanol, Methoxyethanol, Diethylenglykolmonomethylether oder Diethylenglykolmonoethylether.

Das erfindungsgemäße Verfahren (b) wird gegebenenfalls in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Die Hydrierung im erfindungsgemäßen Verfahren (b) kann statt in Gegenwart von Wasserstoff in Kombination mit einem Katalysator auch in Anwesenheit von Triethylsilan durchgeführt werden.

Die Reaktionstemperaturen können bei der Durchführung des erfindungsgemäßen Verfahrens (b) in einem größeren Bereich variiert werden. Im allgemeinen arbeitet man bei Temperaturen von 0°C bis 150°C, vorzugsweise bei Temperaturen von 0°C bis 80°C.

Als Verdünnungsmittel zur Durchführung des erfindungsgemäßen Verfahrens (c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (c) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen von O°C bis 150°C, vorzugsweise bei Temperaturen von 20°C bis 110°C.

Zur Durchführung des erfindungsgemäßen Verfahrens (c) zur Herstellung der Verbindungen der Formel (I) setzt man pro Mol des Pyrazolylcarboxanilids der Formel (Ia) im allgemeinen 0,2 bis 5 Mol, vorzugsweise 0,5 bis 2 Mol an Halogenid der Formel (V) ein.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides,
Rhizoctonia-Arten, wie beispielsweise Rhizoctonia solani.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Puccinia-Arten und von Krankheiten im Wein-, Obst- und Gemüseanbau, wie beispielsweise gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Altemaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Wammebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können alssolche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxychinolinsulfat; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover, Flumorph; Fluoromide; Fluoxastrobin; Fluquinconazole; Flurprimidol; Flusilazole; Flusulfamide; Flutolanil; Flutriafol; Folpet; Fosetyl-Al; Fosetylsodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris-(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; ethfwoxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyc1acis; Tetraconazole; Thiabendazole; Thicyofen; Thifluzamide; Thiophanate-methyl; Thiram; Tioxymid; Tolclofosmethyl; Tolylfluanid; Triadimefon; Triadimenol; Triazbutil; Triazolide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1Naphthalenyl)-1H-pyrrol-2,5-dion; 2,3,5,6-Tetrachlor-4-(methylsulfonyl)-pyridin; 2-Amino-4-methyl-N-phenyl-5-tiazolcarboxamid; 2-Chlor-N-(2,3-dihydro-1,1,3-trimethyl-1H-inden-4-yl)-3-pyridincarboxamide; 3,4,5-Trichlor-2,6-pyridin-dicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylau Monokaliumcarbonat; N-(6-Methoxy-3-pyridinyl)-cyclopropancarboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper, Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chloro benzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine, DDT, Deltamethrin, Demeton-S-methyl, Demeton-S-methylsulphon, Diafenthiwon, Dialifos, Diazinon, Dicldofentlüon, Dichlorvos, Dicofol, Dicrotophos, Dicyclanil, Diflubenzuron, Dimefludirin, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-Gyhalothrin, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilue, Kadethrin, Kempolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluon, Noviflumuron, OK-5101, OK-5201, OK-9601, OK-9602, OK-9701, OK-9802, Omethoate, Oxamyl, Oxydemeton-methyl, Paecilomyces fumosoroseus, Parathion-methyl, Parathion (-ethyl), Permethrin (cis-, trans-), Petroleum, PH-6045, Phenothrin (IRtrans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Telramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb, Vamidothion, Vaniliprole, Verbutin, Verticillium lecanii, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befmdlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Emteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imida1zolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf®) (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellunesbeispiele

### Beispiel 1 (Vergleichsbeispiel)

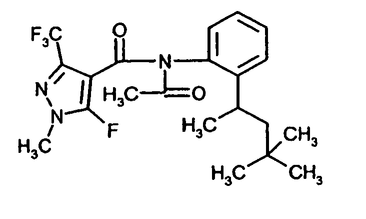

Zu einer Lösung von 300.0 mg (0.78 mmol) 5-Fluor-1-methyl-3-(trifluormethyl)-N-[2-(1,3,3-trimethylbutyl)phenyl]-1H-pyrazol-4-carboxamid in 10.0 ml Tetrahydrofuran werden bei Raumtemperatur 56.0 mg (2.3 mmol) Natriumhydrid gegeben. Die Reaktionsmischung wird 15 min. bei Raumtemperatur gerührt, mit 366.6 mg (4.7 mmol) Acetylchlorid und 2.2 g (21.2 mmol) Essigsäureanhydrid versetzt. Nach 16 h Erhitzen unter Rückfluss wird die Reaktionsmischung auf 100 ml Methanol und 3 ml Triethylamin gegeben, filtriert und im Vakuum aufkonzentriert. Umkristallisation aus Cyclohexan liefert 182 mg (54.7% der Theorie) an N-Acetyl-5-fluor-1-methyl-3-(trifluormethyl)-N-[2-(1,3,3-trimethylbutyl)phenyl]-1H-pyrazol-4-carboxamid mit dem logP (pH 2.3) = 4.63.

### Beispiel 2

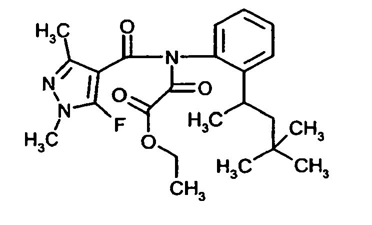

1.66 g (5.0 mmol) 5-Fluor-1,3-dimethyl-N-[2-(1,3,3-trimethylbutyl)phenyl]-1H-pyrazol-4-carboxamid und 1.02 g (7.4 mmol) Ethyl-chlor(oxo)acetat wurden in 20 ml Tetrachlorkohlenstoff gelöst und für 4 h unter Rückfluss gerührt. Das Reaktionsgemisch wurde auf Raumtemperatur abgekühlt und eingeengt. Man erhielt 2 g (93% der Theorie) of Ethyl-{[(5-fluor-1,3-dimethyl-1H-pyrazol-4-yl)carbonyl][2-(1,3,3-trimethylbutyl)phenyl]amino}(oxo)acetat mit dem logP (pH 2.3) = 4.40.

Analog den Beispielen 1 und 2, sowie entsprechend den Angaben in den allgemeinen Verfahrensbeschreibungen, werden die in der nachstehenden Tabelle 1 genannten Verbindungen der Formel (I) erhalten.

**Tabelle 1**

| | | | | | |
|---|---|---|---|---|---|
| 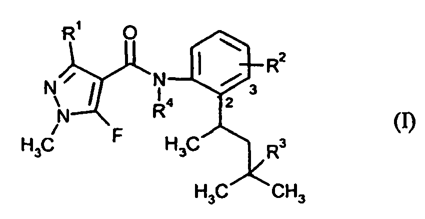 | | | | | |

| **Bsp.** | **R¹** | **R²** | **R**³ | **R⁴** | **logP** |
|---|---|---|---|---|---|
| 3 | -CH₃ | -H | -CH₃ | -CH₃ | 3.64 |
| 4 | -CH₃ | -H | -H | -CH₃ | 3.34 |
| 5 | -CF₃ | -H | -CH₃ | -CH₃ | 4.45 |
| 6 | -CF₃ | -H | -CH₃ | -CH₂-O-CH₃ | 4.60 |

### Herstellune von Auseanesstoffe der Formel (III)

### Beispiel (III-1

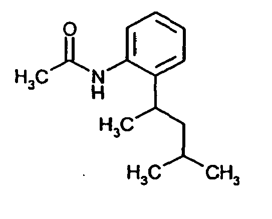

Zu einer Lösung von 500 mg (2.8 mmol) 2-(1,3-Dimethyl-butyl)-phenylamin in 35 ml Acetonitril wurden langsam 243 mg (3.1 mmol) Acetylchlorid gegeben. Das Reaktionsgemisch wurde für 1 h bei Raumtemperatur gerührt und anschließend in Wasser gegossen. Nach Extraktion mit Essigsäureethylester wurden die organischen Phasen über Natriumsulfat getrocknet und unter vermindertem Druck eingeengt. Man erhielt 600 mg (98 % der Theorie) an N-[2-(1,3-Dimethyl-butyl)-phenyl]-acetamid mit dem logP (pH 2.3) = 2.65.

Analog Beispiel (III-1), sowie entsprechend den Angaben in den allgemeinen Verfahrensbeschreibungen, werden die in der nachstehenden Tabelle 2 genannten Verbindungen der Formel (III) erhalten.

**Tabelle 2**

| | | | | |
|---|---|---|---|---|
| 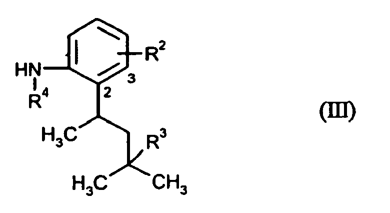 | | | | |

| **Bsp.** | **R²** | **R³** | **R⁴** | **logP** |
|---|---|---|---|---|
| III-2 | -H | -H | -C(=O)H | 2.78 |
| III-3 | -H | -H | -C(=O)CH₂OCH₃ | 3.24 |

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Podosphaera-Test (Apfel) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Podosphaera-Test (Apfel) / protektiv** | | |
|---|---|---|
| Wirkstoff Erfindungsgemäß | Aufwandmenge an Wirkstoff in g/ha | Wirkungsgrad in % |
| 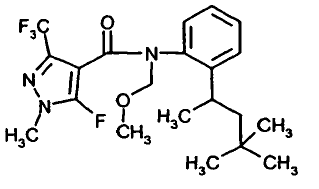 | 100 | 98 |
| 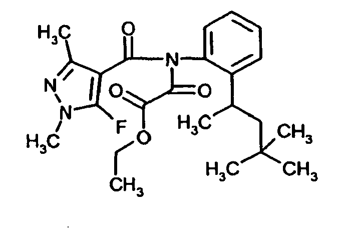 | 100 | 97 |

### Beispiel B

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia - Test (Apfel) / protektiv** | | |
|---|---|---|
| Wirkstoff Erfindungsgemäß | Aufwandmenge an Wirkstoff in g/ha | Wirkungsgrad in % |
| 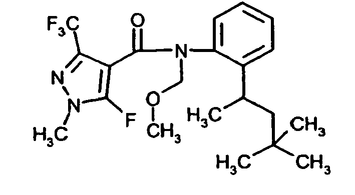 | 100 | 100 |
| 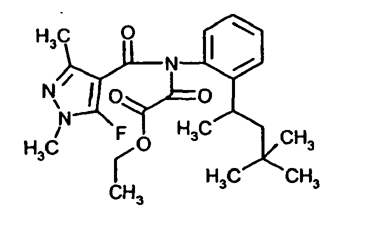 | 100 | 100 |

### Beispiel C

### Botrytis - Test (Bohne) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne) / protektiv** | | |
|---|---|---|
| Wirkstoff Erfindungsgemäß | Aufwandmenge an Wirkstoff in g/ha | Wirkungsgrad in % |
| 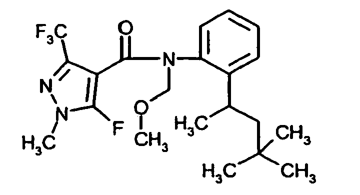 | 500 | 100 |
| 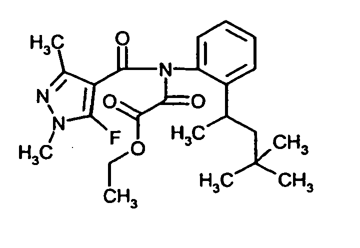 | 500 | 97 |

### Beispiel

### Puccinia-Test (Weizen) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Puccinia-Test (Weizen) / protektiv** | | |
|---|---|---|
| Wirkstoff Erfindungsgemäß | Aufwandmenge an Wirkstoff in g/ha | Wirkungsgrad in % |
| 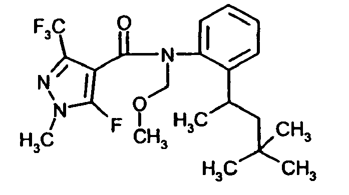 | 500 | 100 |
| 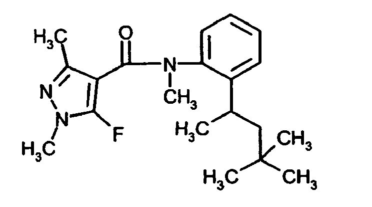 | 500 | 100 |
| 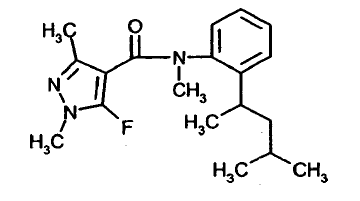 | 500 | 100 |

### Beispiel E

### Sphaerotheca-Test (Gurke) / protektiv

| | |
|---|---|
| Lösungsmittel: | 49 Gewichtsteile N, N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von Sphaerotheca fuliginea inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Sphaerotheca-Test (Gurke) / protektiv** | | |
|---|---|---|
| Wirkstoff Erfindungsgemäß | Aufwandmenge an Wirkstoff in g/ha | Wirkungsgrad in % |
| 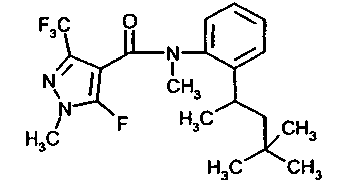 | 750 | 100 |
| 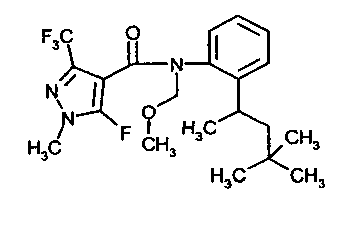 | 750 | 100 |

## Patentansprüche

1. N-substituierte Pyrazolylcarboxamilide der Formel (I) 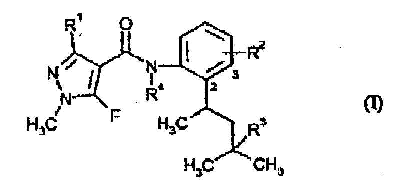 in welcher
R¹ für Methyl steht,
R² für Wasserstoff, Fluor, Chlor, Methyl oder Trifluormethyl steht,
R³ für wasserstoff oder für Halogen, C₁-C₈-Alkyl oder C₁-C₂-Halogenalkyl steht,
R⁴ für C₁-C₈-Alkyl, C₁C₄-Alkoxy-C₁- C₄-alkyl, oder -C(=O)C(=O)R⁵ steht C₄-alkyl,
R⁵ für Wasserstoff, C₁-C₈Alkyl, C₁-C₈-Alkoxy, C₁-C₄-AlkoxyC₁-C₄-alkyl, C₃-C₈- Cycloalkyl; C₁-C₆-Halogenalkyl, C₁-C₆-Halogenalkoxy, Halogen-C₁-C₄-alkoxy-C₁- C₄-alkyl, C₃-C₈-Halogencycloalkyl mit jeweils 1 bis 9 Fluar-, Chor- und/oder ₁ Bromatomen steht.

2. N-substituierte Pyrazolylcarboxanilide der Formel (I) gemäß Anspruch 1, in weicher
R¹ für steht,
R² für Wasserstoff Fluor, Chlor, Methyl oder Trifluormethyl steht,
R³ für Wasserstoff oder für Fluor, Chlor, Brom. Iod, C₁-C₄-Alkyl oder C₁-C₆-Halogenalkyl mit 1 bis 13 Fluor- Chlor- und/oder Bromatomen steht,
R⁴ für C₁-C₆-Alkyl,C₁-C₃-Alkoxy-C₁- C₃-alkyl, oder -C(=O)C(=O)R⁵ steht, C₃-alkyl,
R⁵ für Wasserstoff. C₁-C₆ Alkyl, C₁-C₄-Alkoxy, C₁-C₃-Alkoxy-C₁-C₄-alkyl, C₃-C₆- Cycloalkyl; C₁-C₄-Halogenalkyl, C₁-C₄-Halogenalkoxy, Halogen-C₁-C₃-alkoxy-C₁- C₃-alkyl, C₃-C₆-Halogencycloalkyl mit jeweils 1 bis 9 Fluor-, Chlor- und/oder Bromatomen steht.

3. N-substituierte Pyrazolylcarboxanilide der Formel (Ib) 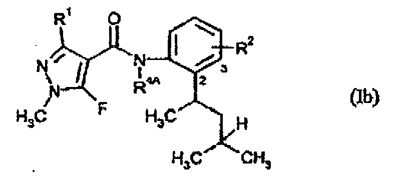 in welcher
R^{4A} für C₁-C₈-Alkyl, C₁-C₄-Alkoxy-C₁-C₄- alkyl, oder -C(=O)C(=O)R⁵ steht, und R¹, R² und R⁵ die in Anspruch 1 angegebenen Bedeutungen haben.

4. N-substituierte Pyrazolylcarboxanilide der Formel (Ic) 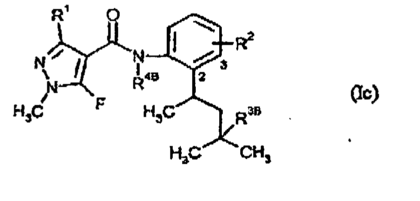 in welcher
R³⁸ für Halogen, C₁-C₈-Alkyl oder C₁-C₈-Halogenalkyl steht,
R^{4B} für C₁-C₈-Alkyl, C₁-C₄-Alkoxy-C₁-C₄- alkyl, oder -C(=O)C(=O)R⁵ steht,
und R¹, R² und R⁵ die in Anspruch 1 angegebenen Bedeutungen haben.

5. N-substituierte Pyrazolylcarboxanilide der Formel (I) gemäß Anspruch I, in welcher R⁴ für Formyl steht.

6. N-substituierte Pyrazolylcarboxanilide der Formel (I) gemäß Anspruch 1, in welcher R⁴ für -C(=O)C(=O)R⁵ steht und R⁵ die in Anspruch 1 angegebenen Bedeutungen hat.

7. Verfahren zum Herstellen der Verbindungen der Formel (I) gemäß Anspruch 1, **dadurch gekennzeichnet, dass** man
a) Carbonsäur-Derivate der Formel (II) 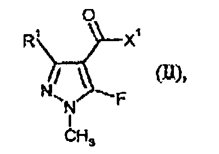 in welcher
R¹ die oben angegebenen Bedeutungen hat und
X¹ für Halogen oder Hydroxy steht,
mit einem Anilinderivat der Formel (III) 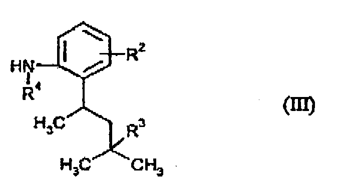 in welcher R³, R³ und R⁴ die oben angegebenen Bedeutungen haben, gegebenenfalls in Gegenwart eines Katalysators, gegebenenfalls in Gegenwart eines Kondensationsmittets, gegebenenfalls in Gegenwart eines Säurebindemittets und gegebenenfalls in Gegenwart eines Verdünnungsmittels umsetzt,
oder
b) Pyrazolylcarboxanilide der Formel (TV) 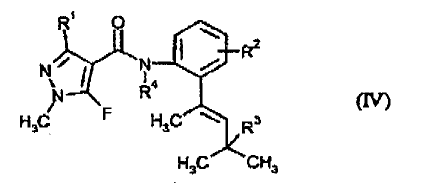 in welcher R¹, R², R³ und R⁴ die oben angegebenen Bedeutungen haben, gegebenenfalls in Gegenwart eines Verdünnungsmittels und gegebenefalls in Gegenwart eines Katalysators hydriert,
oder
c) Pyrazolylcarboxanilide der Formel (Ia) 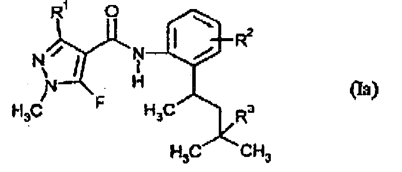 in welcher R¹, R² und R³ die oben angegebenen Bedeutungen haben mit Halogeniden der Formel (V)
R⁴-X² (V)
in welcher
R⁴ die oben angegebenen Bedeutungen hat und
X² für Chlor, Brom oder Iod steht,
in Gegenwart einer Base und in Gegenwart eines Verdünnurigsmittels umsetzt,

8. Mittel zum Bekämpfen unerwünschter Mikroorganismen, **gekennzeichnet durch** einen Gehalt an mindestens einem N-substituiertes Pyrazolylcarboxanilid der Formel (I) gemäß Anspruch 1 neben Streckmitteln und/oder oberflächenaktiven Stoffen.

9. Verwerdung von N-substituierte Pyrazolylcarboxanilide der Formel (I) gemäß Anspruch 1 zum Bekämpfen unerwünschter Mikroorganismen, ausgenommen aus menschlichen oder tierischen körper.

10. Verfahren zum Bekämpfen unerwünschter Mikroorganismem ausgenommen aus menschlichen oder tierisohen körper, **dadurch gekennzeichnet, dass** man N-substituierte Pyrazolylcarboxanilide der Formel (I) gemäß Anspruch 1 auf die Mikroorganismen und/oder deren Lebensraum ausbringt.

11. Verfahren zum Herstellen von Mitteln zum Bekämpfen unerwünschter Mikroorganismen, **dadurch gekennzeichnet, dass** man N-substituterte Pyrazolylcarboxanilide der Formel (I) gemäß Anspruch 1 mit Streckmitteln und/oder oberflächenaktiven Stoffen vermischt.

12. Anilinderivate der Formel (III) 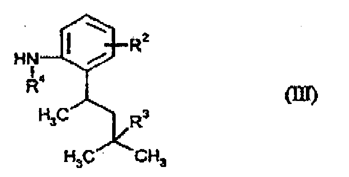 in welcher R², R³ und R⁴ die in Anspruch 1 angegebenen Bedeutungen haben.

13. Pyrazolylcarboxanilide der Formel (IV).

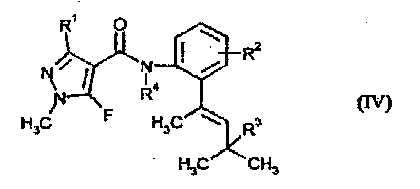

## Claims

1. N-substituted pyrazolylcarboxanilides of the formula (I) 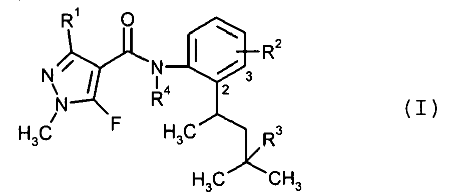 in which
R¹ represents methyl,
R² represents hydrogen, fluorine, chlorine, methyl or trifluoromethyl,
R³ represents hydrogen or represents halogen, C₁- C₈-alkyl or C₁-C₈-haloalkyl,
R⁴ represents C₁-C₈-alkyl, C₁-C₄-alkoxy-C₁-C₄- alkyl, or -C(=O)C(=O)R⁵,
R⁵ represents hydrogen, C₁-C₈-alkyl, C₁-C₈-alkoxy, C₁-C₄-alkoxy-C₁-C₄-alkyl, C₃-C₈-cycloalkyl; C₁- C₆-haloalkyl, C₁-C₆-haloalkoxy, halo-C₁-C₄- alkoxy-C₁-C₄-alkyl, C₃-C₈-halocycloalkyl having in each case 1 to 9 fluorine, chlorine and/or bromine atoms.

2. N-substituted pyrazolylcarboxanilides of the formula (I) according to Claim 1 in which
R¹ represents methyl,
R² represents hydrogen, fluorine, chlorine, methyl or trifluoromethyl,
R³ represents hydrogen, or represents fluorine, chlorine, bromine, iodine, C₁-C₆-alkyl or C₁- C₆-haloalkyl having 1 to 13 fluorine, chlorine and/or bromine atoms,
R⁴ represents C₁-C₆-alkyl, C₁-C₃-alkoxy-C₁-C₃- alkyl, or -C(=O)C(=O)R⁵,
R⁵ represents hydrogen, C₁-C₆-alkyl, C₁-C₄-alkoxy, C₁-C₃-alkoxy-C₁-C₃-alkyl, C₃-C₆-cycloalkyl; C₁- C₄-haloalkyl, C₁-C₄-haloalkoxy, halo-C₁-C₃- alkoxy-C₁-C₃-alkyl, C₃-C₆-halocycloalkyl having in each case 1 to 9 fluorine, chlorine and/or bromine atoms.

3. N-substituted pyrazolylcarboxanilides of the formula (Ib) 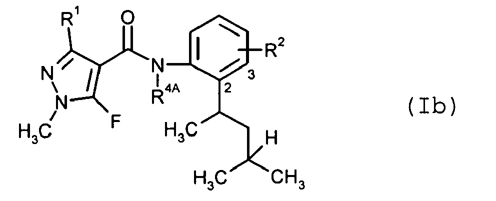 in which
R^{4A} represents C₁-C₈-alkyl, C₁-C₄-alkoxy-C₁-C₄- alkyl, or -C(=O)C(=O)R⁵, and R¹, R² and R⁵ are as defined in Claim 1.

4. N-substituted pyrazolylcarboxanilides of the formula (Ic) 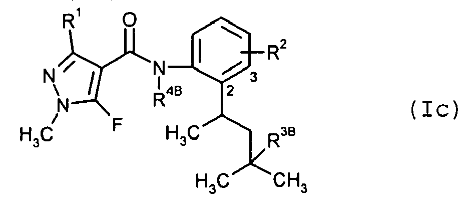 in which
R^{3B} represents halogen, C₁-C₈-alkyl or C₁-C₈- haloalkyl,
R^{4B} represents C₁-C₈-alkyl, C₁-C₄-alkoxy-C₁-C₄- alkyl, or -C(=O)C(=O)R⁵, and R¹, R² and R⁵ are as defined in Claim 1.

5. N-substituted pyrazolylcarboxanilides of the formula (I) according to Claim 1 in which R⁴ represents formyl.

6. N-substituted pyrazolylcarboxanilides of the formula (I) according to Claim 1 in which R⁴ represents -C(=O)C(=O)R⁵ and R⁵ is as defined in Claim 1.

7. Process for preparing compounds of the formula (I) according to Claim 1, **characterized in that**
a) carboxylic acid derivatives of the formula (II) 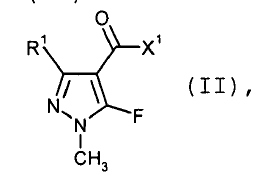 in which
R¹ is as defined above and
X¹ represents halogen or hydroxyl
are reacted with an aniline derivative of the formula (III) 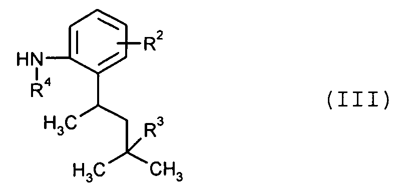 in which R², R³ and R⁴ are as defined above, if appropriate in the presence of a catalyst, if appropriate in the presence of a condensing agent, if appropriate in the presence of an acid binder and if appropriate in the presence of a diluent,
or
b) pyrazolylcarboxanilides of the formula (IV) 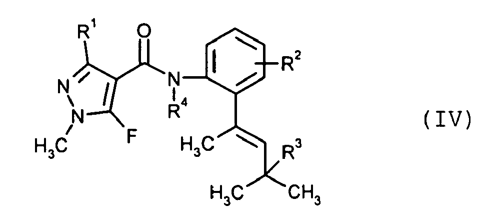 in which R¹, R², R³ and R⁹ are as defined above are hydrogenated, if appropriate in the presence of a diluent and if appropriate in the presence of a catalyst,
or
c) pyrazolylcarboxanilides of the formula (Ia) 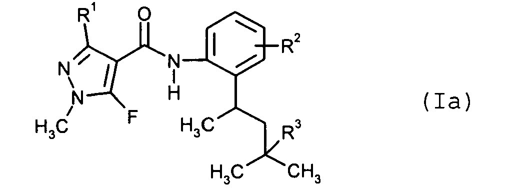 in which R¹, R² and R³ are as defined above are reacted with halides of the formula (V)
R⁴-X² (V)
in which
R⁴ is as defined above and
X² represents chlorine, bromine or iodine
in the presence of a base and in the presence of a diluent.

8. Compositions for controlling unwanted microorganisms, **characterized in that** they comprise at least one N-substituted pyrazolylcarboxanilide of the formula (I) according to Claim 1, in addition to extenders and/or surfactants.

9. Use of N-substituted pyrazolylcarboxanilides of the formula (I) according to Claim 1 for controlling unwanted microorganisms, except for on the human or animal body.

10. Method for controlling unwanted microorganisms, except for on the human or animal body, **characterized in that** N-substituted pyrazolylcarboxanilides of the formula (I) according to Claim 1 are applied to the microorganisms and/or their habitat.

11. Process for preparing compositions for controlling unwanted microorganisms, **characterized in that** N-substituted pyrazolylcarboxanilides of the formula (I) according to Claim 1 are mixed with extenders and/or surfactants.

12. Aniline derivatives of the formula (III) 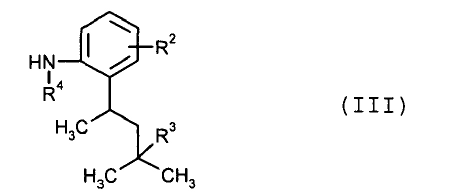 in which R², R³ and R⁴ are as defined in Claim 1.

13. Pyrazolylcarboxanilides of the formula (IV) 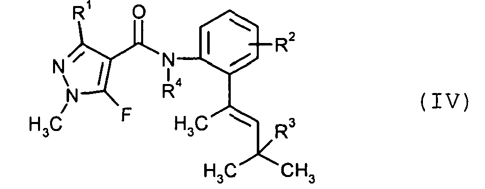 in which R¹, R², R³ and R⁴ are as defined in Claim 1.

## Revendications

1. Pyrazolylcarboxanilides N-substitués de formule (I) 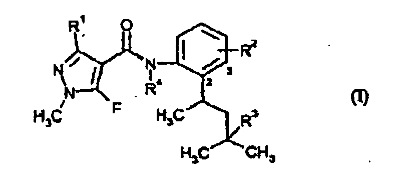 dans laquelle
R¹ représente méthyle,
R² représente hydrogène, fluor, chlore, méthyle ou trifluorométhyle,
R³ représente hydrogène ou halogène, C₁-C₈-alkyle ou C₁-C₈-halogénoalkyle,
R⁴ représente C₁-C₈-alkyle, C₁-C₄-alcoxy-C₁-C₄-alkyle, ou -C(=O)C(=O)R⁵,
R⁵ représente hydrogène, C₁-C₈-alkyle, C₁-C₈-alcoxy, C₁-C₄-alcoxy-C₁-C₄-alkyle, C₃-C₈-cycloalkyle, C₁-C₆- halogénoalkyle, C₁-C₆-halogénoalcoxy, halogène-C₁- C₄-alcoxy-C₁-C₄-alkyle, C₃-C₈-halogénocycloalkyle comprenant à chaque fois 1 à 9 atomes de fluor, de chlore et/ou de brome.

2. Pyrazolylcarboxanilides N-substitués de formule (I) selon la revendication 1, dans laquelle
R¹ représente méthyle,
R² représente hydrogène, fluor, chlore, méthyle ou trifluorométhyle,
R³ représente hydrogène, ou fluor, chlore, brome, iode, C₁-C₆-alkyle ou C₁-C₆-halogénoalkyle comprenant 1 à 13 atomes de fluor, de chlore et/ou de brome,
R⁴ représente C₁-C₆-alkyle, C₁-C₃-alcoxy-C₁-C₃-alkyle, ou -C(=O)C(=O)R⁵,
R⁵ représente hydrogène, C₁-C₆-alkyle, C₁-C₄-alcoxy, C₁-C₃-alcoxy-C₁-C₃-alkyle, C₃-C₆-cycloalkyle, C₁-C₄- halogénoalkyle, C₁-C₄-halogénoalcoxy, halogène-C₁- C₃-alcoxy-C₁-C₃-alkyle, C₃-C₆-halogénocycloalkyle comprenant à chaque fois 1 à 9 atomes de fluor, de chlore et/ou de brome.

3. Pyrazolylcarboxanilides N-substitués de formule (Ib) 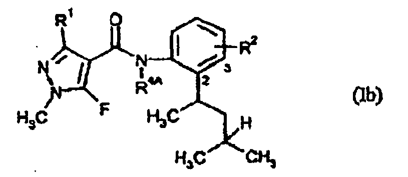 dans laquelle
R^{9A} représente C₁-C₈-alkyle, C₁-C₄-alcoxy-C₁-C₄-alkyle, ou -C(=O)C(=O)R⁵,
et R¹, R² et R⁵ présentent les significations indiquées dans la revendication 1.

4. Pyrazolylcarboxanilides N-substitués de formule (Ic) 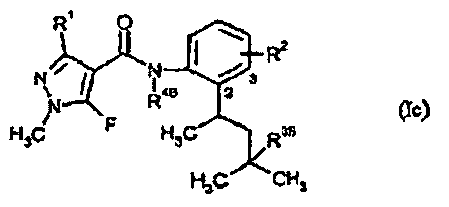 dans laquelle
R^{3B} représente halogène, C₁-C₈-alkyle ou C₁-C₈- halogénoalkyle,
R^{4B} représente C₁-C₈-alkyle, C₁-C₉-alcoxy-C₁-C₉-alkyle, ou -C(=O)C(=O)R⁵,
et R¹, R² et R⁵ présentent les significations indiquées dans la revendication 1.

5. Pyrazolylcarboxanilides N-substitués de formule (I) selon la revendication 1, dans laquelle R⁴ représente formyle.

6. Pyrazolylcarboxanilides N-substitués de formule (I) selon la revendication 1, dans laquelle R⁴ représente -C(=O)C(=O)R⁵ et R⁵ présente les significations indiquées dans la revendication 1.

7. Procédé pour la préparation des composés de formule (I) selon la revendication 1, **caractérisé en ce que**
a) on transforme des dérivés d'acide carboxylique de formule (II), 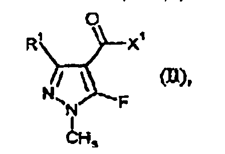 dans laquelle
R¹ présente les significations indiquées ci- dessus et
X¹ représente halogène ou hydroxy,
avec un dérivé d'aniline de formule (III) 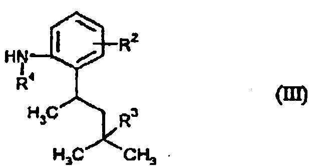 dans laquelle R², R³ et R⁴ présentent les significations indiquées ci-dessus,
le cas échéant en présence d'un catalyseur, le cas échéant en présence d'un agent de condensation, le cas échéant en présence d'un agent liant les acides et le cas échéant en présence d'un diluant,
ou
b) on hydrogène des pyrazolylcarboxanilides de formule (IV) 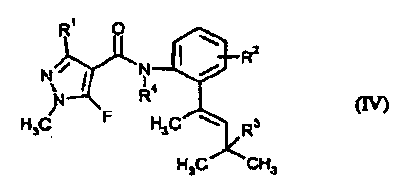 dans laquelle R¹, R², R³ et R⁹ présentent les significations indiquées ci-dessus, le cas échéant en présence d'un diluant et le cas échéant en présence d'un catalyseur,
ou
c) on transforme des pyrazolylcarboxanilides de formule (Ia) 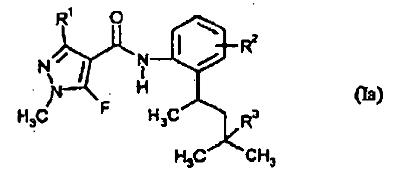 dans laquelle R¹, R² et R³ présentent les significations indiquées ci-dessus, avec des halogénures de formule (V)
R⁴-X² (V)
dans laquelle
R⁴ présente les significations indiquées ci- dessus et
X² représente chlore, brome ou iode,
en présence d'une base et en présence d'un diluant.

8. Agent pour lutter contre des microorganismes non souhaités, **caractérisé par** une teneur en au moins un pyrazolylcarboxanilide N-substitué de formule (I) selon la revendication 1, outre des agents d'allongement et/ou des substances tensioactives.

9. Utilisation de pyrazolylcarboxanilides N-substitués de formule (I) selon la revendication 1 pour lutter contre des microorganismes non souhaités, sauf sur le corps humain ou animal.

10. Procédé pour lutter contre des microorganismes non souhaités, sauf sur le corps humain ou animal, **caractérisé en ce qu'**on applique des pyrazolylcarboxanilides N-substitués de formule (I) selon la revendication 1 sur les microorganismes et/ou leur espace de vie.

11. Procédé pour préparer des agents pour lutter contre des microorganismes non souhaités, **caractérisé en ce qu'**on mélange des pyrazolylcarboxanilides N-substitués de formule (I) selon la revendication 1 avec des agents d'allongement et/ou des substances tensioactives.

12. Dérivés d'aniline de formule (III) 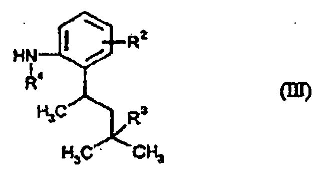 dans laquelle R², R³ et R⁴ ont les significations indiquées dans la revendication 1.

13. Pyrazolylcarboxanilides de formule (IV) 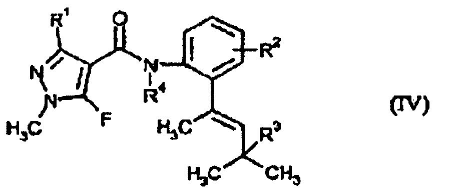 dans laquelle R¹, R², R³ et R⁴ ont les significations indiquées dans la revendication 1.
